# EUROPÄISCHE PATENTSCHRIFT

(11) **EP 0 374 704 B1**
(45) Veröffentlichungstag und Bekanntmachung des Hinweises auf die Patenterteilung: **27.03.1996**
(21) Anmeldenummer: 89122995.7
(22) Anmeldetag: 13.12.1989
(51) Int. Cl.: G03F 7/031, C08F 2/50

(54) **Photopolymerisierbares Gemisch und dieses enthaltendes photopolymerisierbares Aufzeichnungsmaterial**
Photopolymerisable blend and photopolymerisable recording material containing it
Mélange photopolymérisable et matériau d'enregistrement photopolymérisable le contenant

(30) Priorität: 22.12.1988 DE 3843204
(43) Veröffentlichungstag der Anmeldung: 27.06.1990
(73) Patentinhaber: MORTON INTERNATIONAL, INC., Chicago, Illinois 60606 (US)
(72) Erfinder: Steppan, Hartmut, Dr., D-6200 Wiesbaden (DE); Frommeld, Hans-Dieter, Dr., D-6200 Wiesbaden (DE)
(74) Vertreter: Weber, Dieter, Dr.

(56) Entgegenhaltungen:
- EP-A- 0 374 705
- FR-A- 2 095 907
- US-A- 4 587 200

## Beschreibung

Die Erfindung betrifft ein photopolymerisierbares Gemisch, das als wesentliche Bestandteile
a) ein polymeres Bindemittel,
b) eine polymerisierbare Verbindung mit mindestens einer endständigen ethylenischen Doppelbindung und einem Siedepunkt oberhalb 100° C bei Normaldruck und
c) eine 9-Aryl-acridinverbindung als Photoinitiator
enthält.

Aus der DE-C 20 27 467 sind photopolymerisierbare Gemische der vorstehend angegebenen Zusammensetzung bekannt, die als Photoinitiatoren Derivate des Acridins und Phenazins enthalten. Einige Vertreter dieser Verbindungsklasse, z. B. das 9-Phenyl-acridin, zeichnen sich durch eine hohe Lichtempfindlichkeit aus. Die bevorzugten Vertreter haben den Nachteil, daß sie dazu neigen, aus photopolymerisierbaren Schichten, die im Kontakt mit Polyethylenfolien stehen, in diese Folien und durch sie hindurch zu wandern. Dadurch verarmt die Schicht an Initiator und verliert Empfindlichkeit. Auch kann der Initiator aus lichtgehärteten Photoresistschichten in bestimmte Behandlungsbäder, z. B. saure galvanische Bäder, wandern und dort eine störende Gelbfärbung hervorrufen.

Aufgabe der Erfindung war es, photopolymerisierbare Gemische zur Verfügung zu stellen, die eine gleich gute Lichtempfindlichkeit und Bildwiedergabe wie die bevorzugten bekannten Gemische aufweisen, deren Photoinitiatoren aber eine geringere Wanderungstendenz aus der photopolymerisierbaren oder photopolymerisierten Schicht zeigen.

Erfindungsgemäß wird ein photopolymerisierbares Gemisch der eingangs genannten Gattung vorgeschlagen.

Das erfindungsgemäße Gemisch ist dadurch gekennzeichnet, daß die Acridinverbindung der allgemeinen Formel I 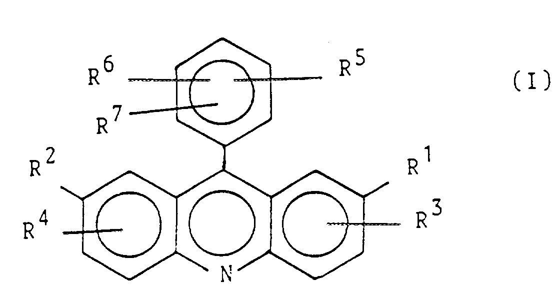 entspricht, worin
- R¹: eine ggf. substituierte Alkyl- oder Acylgruppe bedeutet,
- R, R³ und R⁴: gleich oder verschieden sind und Wasserstoff- oder Halogenatome oder ggf. substituierte Alkyl- oder Acylgruppen bedeuten,
- R⁵, R⁶ und R⁷: gleich oder verschieden sind und Wasserstoff- oder Halogenatome oder ggf. substituierte Alkyl-, Aryl- oder Acylgruppen oder Gruppen der Formel II 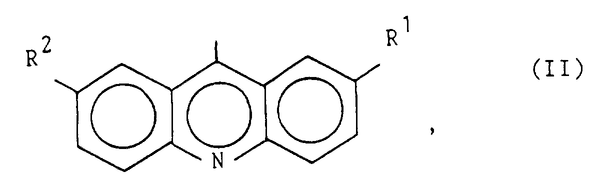
bedeuten.

Erfindungsgemäß wird ferner ein photopolymerisierbares Aufzeichnungsmaterial mit einem Schichtträger und einer darauf aufgebrachten photopolymerisierbaren Schicht vorgeschlagen, die aus dem vorstehend definierten photopolymerisierbaren Gemisch besteht.

In den Verbindungen der allgemeinen Formel I ist R¹ eine Alkylgruppe mit vorzugsweise 1 bis 10 Kohlenstoffatomen, eine aliphatische Acylgruppe mit 2 bis 10 oder eine aromatische Acylgruppe mit 7 bis 15 Kohlenstoffatomen. Diese Gruppen können durch Halogenatome, insbesondere F, Cl oder Br, Hydroxygruppen, Alkoxygruppen, Alkoxycarbonylgruppen, Alkylgruppen, Arylgruppen, Aryloxygruppen, Acylgruppen, Acyloxygruppen, primäre, sekundäre oder tertiäre Aminogruppen, Alkoxycarbonylamino- oder Alkylaminocarbonyloxygruppen substituiert sein. Im allgemeinen hat R¹ ein Molekulargewicht von 15 bis 200, vorzugsweise von 60 bis 150. R ist ein Wasserstoff- oder Halogenatom oder eine Gruppe der Bedeutung von R¹.

Vorzugsweise ist R eine Gruppe der Bedeutung von R¹, wobei R¹ und R gleich oder verschieden sein können.

R³ und R⁴ haben die gleiche allgemeine Bedeutung wie R, vorzugsweise ist mindestens einer dieser Reste ein Wasserstoffatom.

R⁵, R⁶ und R⁷ können die gleiche Bedeutung wie R³ und R⁴ haben und zusätzlich Arylgruppen oder Gruppen der Formel II, insbesondere Halogenatome - vor allem F, Cl oder Br - oder Alkylgruppen, sein. Bevorzugt ist mindestens einer dieser Reste ein Wasserstoffatom.

Die Reste R¹ bis R⁷ haben insgesamt im allgemeinen mindestens 5, bevorzugt 12 bis 40 Kohlenstoffatome. Das Molekulargewicht der Verbindung der Formel I wird durch diese Substituenten gegenüber dem 9-Phenyl-acridin im allgemeinen um 60 bis 800, vorzugsweise um 100 bis 700, insbesondere um 200 bis 600 erhöht.

Verbindungen, in denen mindestens einer der Reste R¹ bis R⁷ eine aromatische Gruppe ist oder enthält, werden bevorzugt. Im allgemeinen ist mindestens einer der Reste R bis R⁷ von Wasserstoff verschieden, vorzugsweise ist es der Rest R. Mit Vorteil werden auch Verbindungen eingesetzt, die in mindestens einem der Substituenten R¹ bis R⁴ ein Halogenatom, insbesondere ein Fluor-, Chlor- oder Bromatom, enthalten. Bevorzugt werden solche Substituenten eingesetzt, die - gegebenenfalls neben der aromatischen Gruppe - mindestens ein Sauerstoffatom enthalten, das ein Ether-, Carbonyl- oder Estersauerstoffatom sein kann.

Die Verbindungen der Formel I können in reiner Form oder im Gemisch untereinander vorliegen und eingesetzt werden, z. B. als Substanzgemische, die bei der Synthese anfallen. Derartige Gemische haben zumeist den Vorteil einer besseren Löslichkeit in Beschichtungslösemitteln als die reinen Verbindungen.

Einige der Verbindungen der Formel I sind bereits bekannt, z. B. das 2,7-Dibenzoyl-9-phenyl-acridin aus J. Org. Chem. 29, 2857 (1964) oder aus J. Chem. Soc. (London) C 1967, 2071 und C 1968, 2900. Eine Anwendbarkeit als Photoinitiatoren ist dort nicht erwähnt.

Die Synthese der Verbindungen der Formel I erfolgt durch Umsetzung von Diphenylamin oder seinen einfachen Substitutionsprodukten, z. B. 4,4′-Dimethyldiphenylamin, 3-Methyldiphenylamin oder 4-Chlordiphenylamin, mit Benzoesäure oder einfachen Benzoesäurederivaten, z. B. t-Butylbenzoesäure, Benzophenon-4-carbonsäure, Diphenyl-4-carbonsäure, 4-Aminomethyl-benzoesäure oder Terephthalsäure in einem geeigneten Reaktionsmedium wie Polyphosphorsäure bei etwa 150 - 200 °C.

Mit Diphenylamin erhält man Acridinverbindungen der Formel I, in denen R¹ eine ggf. substituierte Benzoylgruppe ist, deren Carbonylgruppe umgesetzt, z. B. mit Natriumboranat zur -CHOH-Gruppe reduziert werden kann. Eine Vielzahl von weiteren Derivaten ist in der Folge möglich: Umsetzung der OH-Gruppe mit Säureanhydriden, Isocyanaten, Kondensationsreaktionen mit Phenolen oder Veretherungen.

Beispiele für geeignete Verbindungen der Formel I sind 2,7-Dibenzoyl-9-phenyl-acridin, 2,7-Bis-α-hydroxy-benzyl-9-phenyl-acridin, 2,7-Bis-α-acetoxybenzyl-9-phenyl-acridin, 2,7-Dimethyl-9-(4-methylphenyl)acridin, 2,7-Dimethyl-9-phenyl-acridin, 2,7-Bis-(3,4-dimethyl-benzoyl)-9-(3,4-dimethyl-phenyl)acridin, 2,7-Bis-(α-acetoxy-4-tert.-butyl-benzyl)-9-(4-tert.-butyl-phenyl)acridin, 2,7-Dimethyl-9-(3,4-dichlor-phenyl)acridin, 2,7-Dimethyl-9-(4-benzoyl-phenyl)acridin, 2,7-Bis-(2-chlor-benzoyl)-9-(2-chlor-phenyl)acridin, 2-(α-Hydroxy-3-brom-benzyl)-6-methyl-9-(3-brom-phenyl)acridin, 2,5-Bis-(4-tert.-butyl-benzoyl)-9-(4-tert.-butyl-phenyl)acridin, 1,4-Bis-(2,7-dimethyl-acridin-9-yl)benzol, 2,7-Bis-(α-phenylaminocarbonyloxy-3,4-dimethyl-benzyl)-9-(3,4-dimethylphenyl)acridin und 2,7-Bis-(3,5-dimethyl-4-hydroxy-4′-fluor-diphenylmethyl)-9-(4-fluor-phenyl)acridin.

Der Mengenanteil der Verbindungen der Formel I in dem erfindungsgemäßen Gemisch beträgt im allgemeinen 0,01 bis 10, vorzugsweise 0,1 bis 5 Gew.-%, bezogen auf die nichtflüchtigen Bestandteile.

Für die Zwecke der Erfindung geeignete polymerisierbare Verbindungen sind bekannt und z. B. in den US-A 2 760 863 und 3 060 023 beschrieben.

Bevorzugte Beispiele sind Acryl- und Methacrylsäureester von ein- oder mehrwertigen, bevorzugt mindestens zweiwertigen Alkoholen, wie Ethylenglykoldiacrylat, Polyethylenglykoldimethacrylat, Acrylate und Methacrylate von Trimethylolethan, Trimethylolpropan, Pentaerythrit und Dipentaerythrit und von mehrwertigen alicyclischen Alkoholen oder N-substituierte Acryl- und Methacrylsäureamide. Mit Vorteil werden auch Umsetzungsprodukte von Mono- oder Diisocyanaten mit Partialestern mehrwertiger Alkohole eingesetzt. Derartige Monomere sind in den DE-A 20 64 079, 23 61 041 und 28 22 190 beschrieben. Der Mengenanteil der Schicht an Monomeren beträgt im allgemeinen etwa 10 bis 80, vorzugsweise 20 bis 60 Gew.-%.

Das Gemisch enthält außerdem noch ein polymeres Bindemittel. Als Bindemittel kann eine Vielzahl löslicher organischer Polymerisate Einsatz finden.

Als Beispiele seien genannt: Polyamide, Polyvinylester, Polyvinylacetale, Polyvinylether, Epoxidharze, Polyacrylsäureester, Polymethacrylsäureester, Polyester, Alkydharze, Polyacrylamid, Polyvinylalkohol, Polyethylenoxid, Polydimethylacrylamid, Polyvinylpyrrolidon, Polyvinylmethylformamid, Polyvinylmethylacetamid sowie Mischpolymerisate der Monomeren, die die aufgezählten Homopolymerisate bilden.

Ferner kommen als Bindemittel Naturstoffe oder umgewandelte Naturstoffe in Betracht, z. B. Gelatine und Celluloseether.

Mit besonderem Vorteil werden Bindemittel verwendet, die wasserunlöslich, aber in wäßrig-alkalischen Lösungen löslich oder mindestens quellbar sind, da sich Schichten mit solchen Bindemitteln mit den bevorzugten wäßrigalkalischen Entwicklern entwickeln lassen. Derartige Bindemittel können z. B. die folgenden Gruppen enthalten: -COOH, -PO₃H₂, -SO₃H; -SO₂NH-, -SO₂-NH-SO₂- und -SO₂-NH-CO-.

Als Beispiele hierfür seien genannt: Maleinatharze, Polymerisate aus N-(p-Tolyl-sulfonyl)-carbaminsäure-ß-(methacryloyloxy)-ethylester und Mischpolymerisate dieser und ähnlicher Monomerer mit anderen Monomeren sowie Vinylacetat/Crotonsäure- und Styrol/Maleinsäureanhydrid-Mischpolymerisate. Alkylmethacrylat/Methacrylsäure-Mischpolymerisate und Mischpolymerisate aus Methacrylsäure, höheren Alkylmethacrylaten und Methylmethacrylat und/oder Styrol, Acrylnitril u. a., wie sie in den DE-A 20 64 080 und 23 63 806 beschrieben sind, werden bevorzugt.

Die Menge des Bindemittels beträgt im allgemeinen 20 bis 90, vorzugsweise 40 bis 80 Gew.-% der Bestandteile der Schicht.

Die photopolymerisierbaren Gemische können je nach geplanter Anwendung und je nach den gewünschten Eigenschaften verschiedenartige Stoffe als Zusätze enthalten. Beispiele sind:
Inhibitoren zur Verhinderung der thermischen Polymerisation der Monomeren,
Wasserstoffdonatoren,
die spektrale Lichtempfindlichkeit derartiger Schichten modifizierende Stoffe,
Farbstoffe,
gefärbte und ungefärbte Pigmente,
Farbbildner,
Indikatoren,
Weichmacher, z.B. Polyglykole oder Ester der p-Hydroxybenzoesäure.

Diese Bestandteile sind zweckmäßig so auszuwählen, daß sie in dem für den Initiierungsvorgang wichtigen aktinischen Strahlungsbereich möglichst wenig absorbieren.

Als aktinische Strahlung soll im Rahmen dieser Beschreibung jede Strahlung verstanden werden, deren Energie mindestens der des kurzwelligen sichtbaren Lichts entspricht. Geeignet ist langwellige UV-Strahlung, aber auch Elektronen-, Röntgen- und Laserstrahlung.

Das photopolymerisierbare Gemisch kann für die verschiedensten Anwendungen Einsatz finden, beispielsweise zur Herstellung von Sicherheitsglas, von Lacken, die durch Licht oder Korpuskularstrahlen, z. B. Elektronenstrahlen, gehärtet werden, auf dem Dentalgebiet und insbesondere als lichtempfindliches Aufzeichnungsmaterial auf dem Reproduktionsgebiet.

Die detaillierte Beschreibung der Erfindung beschränkt sich auf dieses Anwendungsgebiet, jedoch ist die Erfindung nicht hierauf beschränkt. Als Anwendungsmöglichkeiten auf diesem Gebiet seien genannt: Aufzeichnungsschichten für die photomechanische Herstellung von Druckformen für den Hochdruck, den Flachdruck, den Tiefdruck, den Siebdruck, von Reliefkopien, z. B. Herstellung von Texten in Blindenschrift, von Einzelkopien, Gerbbildern, Pigmentbildern usw.. Weiter sind die Gemische zur photomechanischen Herstellung von Ätzreservagen, z. B. für die Fertigung von Namensschildern, von kopierten Schaltungen und für das Formteilätzen, anwendbar. Besondere Bedeutung haben die erfindungsgemäßen Gemische als Kopierschichten für die photomechanische Herstellung von Flachdruckformen und für die Photoresisttechnik.

Die gewerbliche Verwertung des Gemischs für die genannten Anwendungszwecke kann in der Form einer flüssigen Lösung oder Dispersion, z. B. als Photoresistlösung, erfolgen, die vom Verbraucher selbst auf einen individuellen Träger, z. B. zum Formteilätzen, für die Herstellung kopierter Schaltungen, von Siebdruckschablonen und dgl., aufgebracht wird. Das Gemisch kann auch als feste lichtempfindliche Schicht auf einem geeigneten Träger in Form eines lagerfähig vorbeschichteten lichtempfindlichen Kopiermaterials, z. B. für die Herstellung von Druckformen, vorliegen, Ebenso ist es für die Herstellung von Trockenresist geeignet.

Es ist im allgemeinen günstig, die Gemische während der Lichtpolymerisation dem Einfluß des Luftsauerstoffes weitgehend zu entziehen. Im Fall der Anwendung des Gemischs in Form dünner Kopierschichten ist es empfehlenswert, einen geeigneten, für Sauerstoff wenig durchlässigen Deckfilm aufzubringen. Dieser kann selbsttragend sein und vor der Entwicklung der Kopierschicht abgezogen werden. Für diesen Zweck sind z. B. Polyesterfilme geeignet. Der Deckfilm kann auch aus einem Material bestehen, das sich in der Entwicklerflüssigkeit löst oder mindestens an den nicht gehärteten Stellen bei der Entwicklung entfernen läßt. Hierfür geeignete Materialien sind z. B. Wachse, Polyvinylalkohol, Polyphosphate, Zucker usw..

Als Schichtträger für mit dem erfindungsgemäßen Gemisch hergestellte Kopiermaterialien sind beispielsweise Aluminium, Stahl, Zink, Kupfer und Kunststoff-Folien, z. B. aus Polyethylenterephthalat oder Cellulosacetat, sowie Siebdruckträger, wie Perlongaze, geeignet.

Die Herstellung der lichtempfindlichen Materialien unter Verwendung des erfindungsgemäßen Gemischs erfolgt in bekannter Weise.

So kann man dieses in einem Lösungsmittel aufnehmen und die Lösung bzw. Dispersion durch Gießen, Sprühen, Tauchen, Antrag mit Walzen usw. auf den vorgesehenen Träger als Film antragen und anschließend antrocknen. Dicke Schichten (z. B. von 250 µm und darüber) werden vorteilhaft durch Extrudieren oder Verpressen als selbsttragende Folie hergestellt, welche dann ggf. auf den Träger laminiert wird. Im Falle von Trockenresist werden Lösungen des Gemischs auf transparente Träger aufgebracht und angetrocknet. Die lichtempfindlichen Schichten - Dicke etwa zwischen 10 und 100 µm - werden dann gleichfalls zunächst durch Laminieren zusammen mit dem temporären Träger auf das gewünschte Substrat auflaminiert.

Die Verarbeitung der Materialien wird in bekannter Weise vorgenommen. Zur Entwicklung werden sie mit einer geeigneten Entwicklerlösung, bevorzugt einer schwach alkalischen wäßrigen Lösung, behandelt, wobei die unbelichteten Anteile der Schicht entfernt werden und die belichteten Bereiche der Kopierschicht auf dem Träger zurückbleiben.

Die erfindungsgemäßen Aufzeichnungsmaterialien zeichnen sich durch einen geringeren Lichtempfindlichkeitsverlust beim Lagern aus. Dieser Vorteil wird offenbar durch eine höhere Diffusionsfestigkeit der Initiatoren in der photopolymerisierbaren Schicht gegenüber dem unsubstituierten 9-Phenyl-acridin bewirkt. Die Diffusionsfestigkeit steigt mit zunehmendem Molekulargewicht. Dabei ist es wesentlich, daß die Substituenten in der 2- oder bevorzugt in 2,7-Stellung des Acridinkerns stehen. Auch aus der lichtgehärteten Schicht wandern die Initiatoren nicht oder in wesentlich geringerem Maße aus als bekannte Initiatoren.

Im folgenden werden Beispiele für das erfindungsgemäße Gemisch angegeben. Dabei wird zunächst die Herstellung von Verbindungen der Formel I beschrieben. Anschließend werden in Tabelle I Photoinitiatoren angegeben, die in den photopolymerisierbaren Gemischen der Anwendungsbeispiele eingesetzt worden sind.

In den Beispielen stehen Gewichtsteile (Gt) und Volumenteile (Vt) im Verhältnis von g zu ccm. Sofern nicht anders angegeben, verstehen sich Prozent- und Mengenverhältnisse in Gewichtseinheiten.

### Herstellungsbeispiele

### 1. 2,7-Dibenzoyl-9-phenylacridin (Verbindung 1)

1 Gt Diphenylamin und 15 Gt Polyphosphorsäure werden unter Rühren auf 100 °C erwärmt. Nach Zugabe von 2,5 Gt Benzoesäure wird das Gemisch 45 Minuten weiter auf 200 °C erhitzt. Nach dem Abkühlen auf 100 °C wird die Mischung in 70 Gt Wasser gegossen, das Produkt abfiltriert und gereinigt (Fp 210 °C).

### 2. 2,7-Bis-(α-hydroxybenzyl)-9-phenylacridin (Verbindung 2)

1 Gt 2,7-Dibenzoyl-9-phenylacridin wird in 4 Gt Ethanol suspendiert und durch portionsweise Zugabe von 0,1 Gt Natriumboranat bei 20 - 50 °C reduziert. Nach 24 Stunden wird das Reaktionsprodukt mit Wasser ausgefällt, gereinigt und getrocknet (Fp oberhalb 280 °C).

### 3. 2,7-Bis-(α-acetoxybenzyl)-9-phenylacridin (Verbindung 3)

1 Gt 2,7-Bis-(α-hydroxybenzyl)-9-phenylacridin wird in 4 Gt Aceton suspendiert und mit 0,8 Gt Acetanhydrid und 0,001 Gt 4-Dimethylamino-pyridin 1 Stunde unter Rückfluß erhitzt. Das Gemisch wird danach mit Wasser versetzt und das Produkt abfiltriert.

### 4. 2,7-Dimethyl-9-(p-tolyl)-acridin (Verbindung 4)

1 mol 4,4′-Dimethyldiphenylamin und 1 mol 4-Methylbenzoesäure werden in 5000 g Polyphosphorsäure 1 Stunde auf 200 °C erhitzt. Nach Abkühlen wird das Reaktionsgemisch mit Wasser und Ammoniak versetzt, das Produkt abgesaugt und gereinigt (Fp 210 - 211 °C).

**Tabelle**

| Verbindungen der Formel I mit R³ = R⁴ = R⁷ = H | | | | |
|---|---|---|---|---|
| Verbindung | R¹ | R | R⁵ | R⁶ |
| 1 | Benzoyl | Benzoyl | H | H |
| 2 | α-Hydroxy-benzyl | α-Hydroxy-benzyl | H | H |
| 3 | α-Acetoxy-benzyl | α-Acetoxy-benzyl | H | H |
| 4 | Methyl | Methyl | 4-Methyl | H |
| 5 | Methyl | Methyl | H | H |
| 6 | 3,4-Dimethyl-benzoyl | 3,4-Dimethyl-benzoyl | 4-Methyl | 3-Methyl |
| 7 | α-Acetoxy-p-tert.-butyl-benzyl | α-Acetoxy-p-tert.-butyl-benzyl | 4-tert.-butyl | H |

### Anwendungsbeispiele

### Beispiel 1

Die folgenden 3 Beschichtungslösungen wurden hergestellt aus
- 50 Gt: eines Terpolymerisats aus Methylmethacrylat, n-Hexylmethacrylat und Methacrylsäure (5:60:35) mit dem mittleren Molekulargewicht M_{w} = 70.000,
- 11 Gt: des Diurethans aus 2 mol Hydroxyethylmethacrylat und 1 mol 2,2,4-Trimethylhexamethylendiisocyanat,
- 39 Gt: des Umsetzungsprodukts aus 1 mol Hydroxyethylacrylat, 2 mol Caprolacton und 1 mol n-Butylisocyanat,
- 0,1 Gt: des blauen Farbstoffs 1,4-Bis-isobutylaminoanthrachinon (C.I. 61551),
- 160 Gt: Butanon und
- 40 Gt: Ethanol,
denen als Photoinitiator
a 0,51 Gt 9-Phenylacridin (MG 255; Vergleich)
b 0,926 Gt Verbindung 1 (MG 464)
c 1,102 Gt Verbindung 3 (MG 552)
zugesetzt wurden.

Die Lösungen wurden auf biaxial verstreckte und thermofixierte Polyethylenterephthalatfolien von 25 µm Stärke so aufgebracht, daß nach dem Trocknen bei 100°C jeweils ein Schichtgewicht von 45 g/m erhalten wurde.

Um die Trockenresistschichten vor Verschmutzung durch Staub und vor Beschädigungen zu schützen, wurden sie mit einer 23 µm starken Deckfolie aus Polyethylen kaschiert und aufgerollt. Danach können sie über einen längeren Zeitraum unter Lichtausschluß gelagert werden. Die Rollen (Polyethylenfolie nach außen) wurden mit einem handelsüblichen Klebeband fixiert.

Nach 24-stündiger Lagerung der Rollen bei 40 °C wurden die Resists verarbeitet.

Beim Abziehen des Klebebands zeigte sich, daß das Band, obwohl durch die Polyethylen-Deckfolie vom Resist getrennt, durch diffundierten Anthrachinonfarbstoff hellblau gefärbt war. Um zu prüfen, ob das Klebeband auch Spuren der Photoinitiatoren enthielt, wurde mit einem Spektrophotometer, Typ Perkin-Elmer Lambda 3, ein UV-Spektrum bei 350 - 450 nm aufgenommen. Das Klebeband des Musters a zeigte im Vergleich zu einem Vergleichsmuster einen Peak (Extinktion = 0,12) bei 358 nm. Die hellblauen Klebebänder b und c zeigten keine zusätzliche Extinktion zwischen 350 und 450 nm. Sie enthielten keinen Photoinitiator.

Die Trockenresistfolien wurden in einer handelsüblichen Laminiervorichtung bei 115 °C auf mit 35 µm starker Kupferfolie kaschierte Phenoplast-Schichtstoffplatten laminiert und mittels einer 5-kW-Metallhalogenidlampe im Abstand von 110 cm zwichen Lampe und Vakuumkopierrahmen 4 Sekunden belichtet. Als Vorlage diente ein 13-stufiger Belichtungskeil, der Dichteinkremente von 0,15 enthielt. Dabei wurde der Belichtungskeil so plaziert, daß er auf den Teilen lag, die ursprünglich von Polyethylenfolie und Klebeband bedeckt gewesen waren.

Nach der Belichtung wurden die Polyesterfolien abgezogen und die Schichten mit 1 %iger Natriumcarbonatlösung in einem Sprühentwicklungsgerät innerhalb 60 s entwickelt.

Die Probe a war unterbelichtet, der Resist war nur bis zur Stufe 5 ausgehärtet; Linien wurden um 10 % zu schmal wiedergegeben. Der Stufenkeil der Muster b und c war dagegen bis zur Stufe 6 voll ausgehärtet. Eine Testvorlage mit 80 µm breiten hellen und dunklen Linien wurde maßstabgetreu als Reliefbild wiedergegeben.

### Beispiel 2

In Beschichtungslösungen aus
- 40 Gt: eines Copolymerisats aus Methylmethacrylat und Methacrylsäure (Säurezahl 115),
- 40 Gt: Trimethylolpropantriacrylat und
- 0,5 Gt: Disperse Blue 134 (C.I. 61551) in
- 520 Gt: 2-Methoxyethanol
wurden als Photoinitiatoren
a 0,70 Gt 9-(3,4-Dimethyl-phenyl)-acridin (MG 283, Vergleich)
b 0,70 Gt Verbindung 5 (MG 283)
c 1,35 Gt Verbindung 6 (MG 547)
gelöst. Die Lösungen wurden auf elektrolytisch aufgerauhtes und durch Anodisierung gehärtetes 0,3 mm starkes Aluminium durch Aufschleudern aufgetragen. Die Schicht wurde 2 Minuten bei 100 °C getrocknet, wobei man ein Schichtgewicht von 2,4 g/m erhielt.

Die beschichteten, leicht klebrigen lichtempfindlichen Platten wurden zum Schutz gegen Verschmutzung bei Raumtemperatur mit einer 20 µm starken Polyethylenfolie kaschiert.

Die so erhaltenen Druckplatten wurden mit einer 5-kW-Metallhalogenidlampe 15 Sekunden in einem Abstand von 110 cm unter einer Negativvorlage zusammen mit einem 13-stufigen Belichtungskeil mit Dichteinkrementen von 0,15 belichtet.

Die durch Licht nicht gehärteten Teile wurden durch Überwischen mit einer Entwicklerlösung folgender Zusammensetzung entfernt:
- 3 Gt: Natriummetasilikat x 9 H₂O,
- 0,05 Gt: Strontiumchlorid,
- 0,03 Gt: nichtionogenes Netzmittel (Kokosfettalkohol-Polyoxyethylenether mit ca. 8 Oxyethyleneinheiten),
- 0,003 Gt: Antischaummittel,
- 100 Gt: vollentsalztes Wasser.

Die Zahl der voll durchgehärteten Stufen des Belichtungskeils gibt eine Maßzahl für die Lichtempfindlichkeit an. Die Werte sind in der folgenden Tabelle unter (A) zusammengestellt.

Um die Diffusion des Initiators durch die Polyethylenfolie zu verdeutlichen, wurde auf je einer Musterplatte auf die Polyethylenfolie ein handelsübliches farbloses transparentes Klebeband gedrückt und der Verbund 24 Stunden bei Raumtemperatur (23 °C) gelagert. Danach wurde die Klebefolie wieder abgezogen und (B) die Erhöhung der optischen Dichte bei 358 nm gemesen. Weiter wurde (Versuch C) an die Stelle der entfernten Klebefolie der Belichtungstestkeil mit 13 Graustufen gelegt und die Druckplatte ebenso wie oben 15 Sekunden belichtet und dann entwickelt.

Die in der folgenden Tabelle zusammengefaßten Ergebnisse zeigen, daß der Initiator aus dem Muster a) am stärksten diffundiert, aus Muster b deutlich weniger und aus Muster c nicht diffundiert.

| | (A) Keilstufen | (B) Dichtedifferenz | (C) Keilstufen |
|---|---|---|---|
| a | 6 | 0,17 | 2 |
| b | 7 | 0,11 | 4 |
| c | 7 | 0 | 7 |

### Beispiel 3

Beschichtungslösungen aus
- 65 Gt: eines Copolymeren aus Methylmethacrylat, Butylacrylat, Styrol und Methacrylsäure (35:40:5:20),
- 35 Gt: Trimethylolpropantriacrylat,
- 1 Gt: Leukokristallviolett und
- 0,05 Gt: Kristallviolett in
- 75 Gt: Butanon und
- 75 Gt: Ethanol
wurden als Initiator
a 1 Gt Verbindung 7 oder
b 1 Gt Isopropylthioxanthon und
2 Gt p-Dimethylamino-benzoesäureester (Initiatormischung eines handelsüblichen Photoresists)
zugesetzt.

Die Lösungen wurden wie in Beispiel 1 auf Polyethylenterephthalatfolie aufgebracht, getrocknet, auf Kupferfolie laminiert, belichtet und entwickelt. Dann wurde die optimale Belichtungszeit ermittelt.

In einem weiteren Versuch wurden wie oben hergestellte und entwickelte Platten 30 s mit Leitungswasser gespült, 30 s in einer 15 %igen Ammoniumperoxydisulfat-Lösung angeätzt, erneut mit Wasser gespült, 30 s in 10 %ige Schwefelsäure getaucht und sodann nacheinander in den folgenden Elektrolytbädern galvanisiert.
1. 60 Minuten in einem Kupferlektrolytbad der Firma Schlötter, Geislingen/Steige, Typ "Glanzkupfer-Bad"
   Stromdichte: 2,5 A/dm
   Metallaufbau: ca. 30 µm
   Temperatur: Raumtemperatur
2. 15 Minuten in einem Bleizinnbad LA der Firma Schlötter, Geislingen/Steige
   Stromdichte: 2 A/dm
   Metallaufbau: 15 µm
   Temperatur: Raumtemperatur

Die Platten zeigten keinerlei Unterwanderungen oder Beschädigungen.

Zur Prüfung, ob Resistbestandteile in die Galvanobäder diffundieren, wurden Glasplatten mit 0,075 m Resist beschichtet und eine Stunde in 1 Liter 20 %ige Schwefelsäure getaucht. In beiden Fällen blieb die Schwefelsäure farblos: Von beiden Schwefelsäureauszügen wurden danach mit dem Spektrophotometer (1 cm Küvette) ein UV-Spektrum (200 - 800 nm) aufgenommen, b) zeigte einen deutlichen Peak bei 225 nm (Extinktion = 0,95), Teile des Initiatorsystems sind in die Schwefelsäure gewandert; a) zeigte keine Verunreinigung.

## Patentansprüche

1. Photopolymerisierbares Gemisch, das als wesentliche Bestandteile
a) ein polymeres Bindemittel,
b) eine polymerisierbare Verbindung mit mindestens einer endständigen ethylenischen Doppelbindung und einem Siedepunkt oberhalb 100° C bei Normaldruck und
c) eine 9-Aryl-acridinverbindung als Photoinitiator
enthält, dadurch gekennzeichnet, daß die Acridinverbindung der allgemeinen Formel I 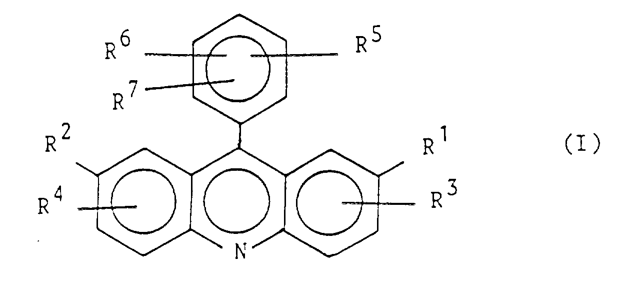 entspricht, worin
R¹ eine ggf. substituierte Alkyl- oder Acylgruppe bedeutet,
R, R³ und R⁴ gleich oder verschieden sind und Wasserstoff- oder Halogenatome oder ggf. substituierte Alkyl- oder Acylgruppen bedeuten,
R⁵, R⁶ und R⁷ gleich oder verschieden sind und Wasserstoff- oder Halogenatome oder ggf. substituierte Alkyl-, Aryl- oder Acylgruppen oder Gruppen der Formel II 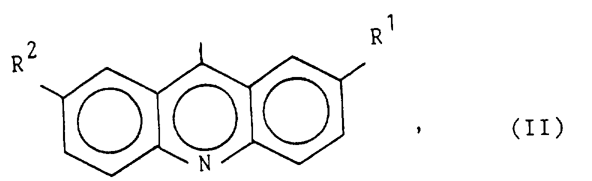
bedeuten.

2. Photopolymerisierbares Gemisch nach Anspruch 1, dadurch gekennzeichnet, daß R³, R⁴ und R⁵ Wasserstoffatome sind.

3. Photopolymerisierbares Gemisch nach Anspruch 1, dadurch gekennzeichnet, daß die Reste R¹ bis R⁷ zusammen mindestens 6 Kohlenstoffatome enthalten.

4. Photopolymerisierbares Gemisch nach Anspruch 1, 2 oder 3, dadurch gekennzeichnet, daß mindestens einer der Reste R¹ bis R⁷ eine aromatische Gruppe enthält.

5. Photopolymerisierbares Gemisch nach Anspruch 1, dadurch gekennzeichnet, daß mindestens einer der Reste R bis R⁷ von Wasserstoff verschieden ist.

6. Photopolymerisierbares Gemisch nach Anspruch 1, dadurch gekennzeichnet, daß die Reste R¹ bis R⁷ zusammen das Molekulargewicht der Verbindung der Formel I um 100 bis 700 erhöhen.

7. Photopolymerisierbares Gemisch nach Anspruch 1, dadurch gekennzeichnet, daß mindestens einer der Reste R¹ bis R⁷ ein Sauerstoffatom enthält.

8. Photopolymerisierbares Gemisch nach Anspruch 1, dadurch gekennzeichnet, daß das Bindemittel wasserunlöslich und in wäßrig-alkalischen Lösungen löslich ist.

9. Photopolymerisierbares Gemisch nach Anspruch 1, dadurch gekennzeichnet, daß die polymerisierbare Verbindung ein Ester der Acryl- oder Methacrylsäure mit einem aliphatischen Alkohol ist.

10. Photopolymerisierbares Aufzeichnungsmaterial mit einem Schichtträger und einer photopolymerisierbaren Schicht, dadurch gekennzeichnet, daß die Schicht aus einem Gemisch gemäß einem der Ansprüche 1 bis 9 besteht.

## Claims

1. Photopolymerizable mixture which contains, as essential components,
a) a polymeric binder,
b) a polymerizable compound having at least one terminal ethylenic double bond and a boiling point above 100°C at atmospheric pressure and
c) a 9-arylacridine compound as a photoinitiator,
characterized in that the acridine compound corresponds to the general formula I 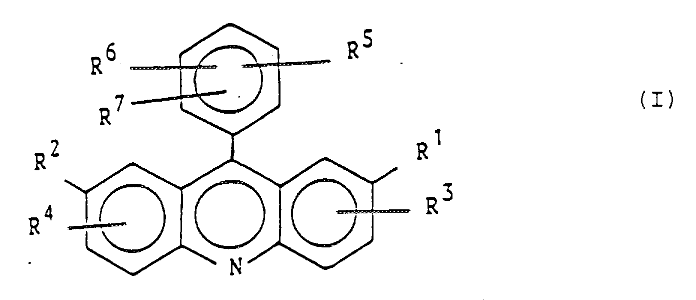 in which
R¹ denotes an optionally substituted alkyl or acyl group,
R, R³ and R⁴ are identical or different and denote hydrogen or halogen atoms or optionally substituted alkyl or acyl groups and
R⁵, R⁶ and R⁷ are identical or different and denote hydrogen or halogen atoms or optionally substituted alkyl, aryl or acyl groups or groups of the formula II

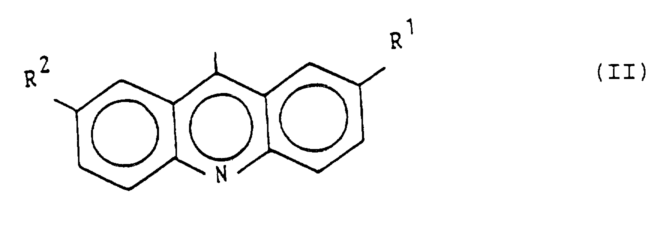

2. Photopolymerizable mixture according to Claim 1, characterized in that R³, R⁴ and R⁵ are hydrogen atoms.

3. Photopolymerizable mixture according to Claim 1, characterized in that the radicals R¹ to R⁷ together contain at least 6 carbon atoms.

4. Photopolymerizable mixture according to Claim 1, 2 or 3, characterized in that at least one of the radicals R¹ to R⁷ contains an aromatic group.

5. Photopolymerizable mixture according to Claim 1, characterized in that at least one of the radicals R to R⁷ is not hydrogen.

6. Photopolymerizable mixture according to Claim 1, characterized in that the radicals R¹ to R⁷ together increase the molecular weight of the compound of the formula I by from 100 to 700.

7. Photopolymerizable mixture according to Claim 1, characterized in that at least one of the radicals R¹ to R⁷ contains an oxygen atom.

8. Photopolymerizable mixture according to Claim 1, characterized in that the binder is water-insoluble and soluble in aqueous alkaline solutions.

9. Photopolymerizable mixture according to Claim 1, characterized in that the polymerizable compound is an ester of acrylic or methacrylic acid with an aliphatic alcohol.

10. Photopolymerizable recording material having a substrate and a photopolymerizable layer, characterized in that the layer consists of a mixture according to any of Claims 1 to 9.

## Revendications

1. Mélange photopolymérisable, qui contient comme constituants essentiels :
a) un liant polymère,
b) un composé polymérisable comportant au moins une double liaison éthylénique terminale et ayant un point d'ébullition supérieur à 100°C sous la pression normale, et
c) une 9-arylacridine comme composé photoamorceur,
mélange caractérisé en ce que l'acridine est un composé répondant à la formule générale I: 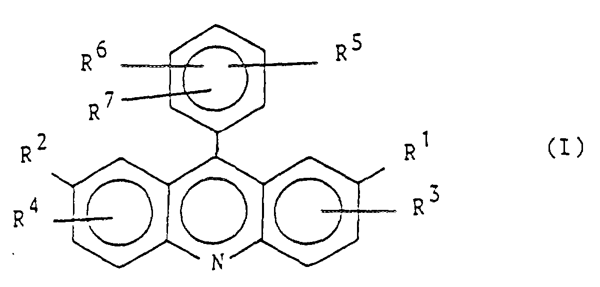 dans laquelle
R¹ représente un groupe alkyle ou acyle éventuellement substitué,
R, R³ et R⁴ sont identiques ou différents et représentent chacun un atome d'hydrogène ou d'halogène ou un groupe alkyle ou acyle éventuellement substitué,
R⁵, R⁶ et R⁷ sont identiques ou différents et représentent chacun un atome d'hydrogène ou d'halogène ou un groupe alkyle, aryle ou acyle éventuellement substitué ou un groupe de formule II :

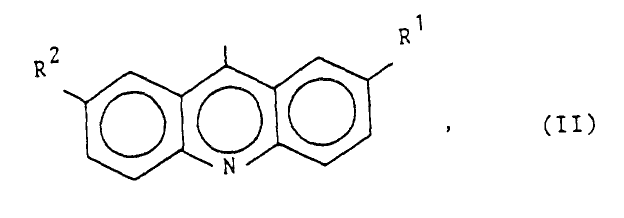

2. Mélange photopolymérisable selon la revendication 1, caractérisé en ce que R³, R⁴ et R⁵ représentent chacun un atome d'hydrogène.

3. Mélange photopolymérisable selon la revendication 1, caractérisé en ce que les restes R¹ à R⁷ contiennent ensemble au moins 6 atomes de carbone.

4. Mélange photopolymérisable selon la revendication 1, 2 ou 3, caractérisé en ce qu'au moins l'un des restes R¹ à R⁷ contient un groupe aromatique.

5. Mélange photopolymérisable selon la revendication 1, caractérisé en ce qu'au moins l'un des restes R à R⁷ est différent de l'hydrogène.

6. Mélange photopolymérisable selon la revendication 1, caractérisé en ce que les restes R¹ à R⁷ élèvent d'au moins 100 à 700 le poids moléculaire du composé de formule I.

7. Mélange photopolymérisable selon la revendication 1, caractérisé en ce qu'au moins l'un des restes R¹ à R⁷ contient un atome d'oxygène.

8. Mélange photopolymérisable selon la revendication 1, caractérisé en ce que le liant est insoluble dans l'eau et est soluble dans des solutions alcalines aqueuses.

9. Mélange photopolymérisable selon la revendication 1, caractérisé en ce que le composé polymérisable est un ester de l'acide acrylique ou de l'acide méthacrylique avec un alcool aliphatique.

10. Matériau d'enregistrement photopolymérisable, comportant un support de couche et une couche photopolymérisable, caractérisé en ce que la couche consiste en un mélange selon l'une des revendications 1 à 9.
